# EUROPEAN PATENT SPECIFICATION

(11) **EP 2 542 188 B1**
(45) Date of publication and mention of the grant of the patent: **02.11.2016**
(21) Application number: 11710579.1
(22) Date of filing: 11.02.2011
(51) Int. Cl.: A61F 2/44, A61F 2/46

(54) **PROSTHESIS FOR SPINAL COLUMN**
WIRBELSÄULENPROTHESE
PROTHÈSE DE COLONNE VERTÉBRALE ANTÉRIEURE

(30) Priority: 03.03.2010 PL 39060110
(43) Date of publication of application: 09.01.2013
(73) Proprietor: LFC Spólka Z.O.O., 65-364 Zielona Góra (PL)
(72) Inventor: CIUPIK, Lechos aw, Franciszek, 65-364 Zielona Góra (PL); POWCHOWICZ, Pawel, 65-980 Zielona Góra (PL); ASHKENAZI, Ely, 96431 Jerusalem (IS)
(74) Representative: Theobald, Andreas
(86) International application number: PCT/PL2011/000013
(87) International publication number: WO 2011/108950

(56) References cited:
- EP-A1- 1 790 301
- EP-A1- 1 925 271
- WO-A1-99/12481
- WO-A1-2010/045231
- WO-A2-2006/058018
- WO-A2-2007/124352
- WO-A2-2010/075555
- DE-A1- 4 328 690
- US-A1- 2003 004 576
- US-A1- 2006 030 857
- US-A1- 2008 221 694

## Description

The invention relates to a prosthesis of an anterior spinal column, prosthesis-guiding instrument and method for installation thereof, which find application in the treatment of cervical, thoracic and lumbar spinal segments.

From the patent application US2009/0149955 a spinal prosthesis is known in the form of a cylinder with a perforated wall and prostheses of intervertebral discs attached to it made of spring silicone rubber in the shape of balloons. Anchoring of the cylinder in the damaged vertebra is provided by means of an entrapment of an elongate lug of the cylinder within a slot of a plate retained by screws within a recess of the damaged vertebra. The springs of the resilient beads are attached to the natural vertebrae, superior and inferior to the damaged vertebra by fixing plates provided with flanges that are fixed by screws to those vertebrae. Where adjoining vertebrae are damaged, two or more prosthetic cylinders for anchoring to a single vertebra are used with interconnecting resilient beads. The inconvenience of this solution is its complex structure and the necessity of boring into vertebral bone during installation of the prosthesis and fixing it with additional screws.

From the patent application US2006/064168 a prosthesis for a partial replacement of a vertebral body is known, which has an upper contact plate for connection to an upper vertebral body, a lower contact plate for connection to a lower vertebral body, and a bridging part which connects the upper and lower contact plates to each other and bridges at least one vertebral body which is located between the upper and lower vertebral bodies. The bridging part is accommodated in a recess in the vertebral body. To be secured in said recess, the prosthesis has lateral anchoring projections, which penetrate into the bone substance, located on both sides of the bridging part. Its cross section narrows toward the rear preferably in a trapezoid shape. The inconvenience of this solution is the complex structure of the prosthesis and necessity of accommodating the bony recess to the bridging part to reinforce the support of the vertebral body.

A prosthesis for the replacement of all vertebral elements such as the vertebral body, the pedicles with the joint facets and the lamina is known from the patent application EP1188424. The prosthesis has interconnection capability to a posterior fusion system by means of special screws. The prosthesis consists of the front part, which in a section is in the octagonal shape and screws that can connect it to the posterior fixation pedicle screw system and thus create a complete construction which replaces the whole dislocated vertebral unit. The inconvenience of this solution is the necessity of cooperation with an additional posterior spinal stabilization by a posterior fusion system and the complicated two-stage surgical operation - first, using the posterior, and then the anterio-lateral approach.

A prosthesis which has a telescopic perforated cylinder is known from the patent application EP 0968692 The cylinder consists of two parts and an intersected shield, connected by screws. The telescopic construction allows regulating the length of the prosthesis depending on the distance between the neighboring vertebrae. The prosthesis is situated in a definite position by two external screws fixed in the cylinder's hole. The cylinder is provided with teeth and other anchoring elements preventing sliding of the prosthesis and a decrease of its height during implantation. The inconvenience of this solution is the multi-element construction of the prosthesis. Bearing surfaces of the prosthesis are small, which decreases its load bearing capacity. The round shape of cylinders is not accommodated to the anatomy of the spine. For a proper placement of the prosthesis it is necessary to use an external element in the form of the above mentioned screws.

The inconvenience of the solutions described above is the long duration of surgical implantation of the prosthesis between adjacent vertebrae, the necessity of its fastening with additional screws, and in some cases the necessity of additional stabilization, which complicates the surgery and prolongs implantation time.

A monorail system, which improves the use of the procedure of a spinal fusion, and a method for preparation of the intervertebral space and for introduction of the implant are known from the patent application US 2007/0270873. The system consists of an instrument for distraction of the intervertebral space, preferably in the form of a rod having a rail, chisel provided with a canal cooperating with the rail and an implant provided with a canal cooperating with the rail. The instrument for distraction, whose sliding section is constituted by the rail, cooperates with other instruments used for preparation of the intervertebral space and may act as an instrument for inserting the implant into the intervertebral space. The instrument and the implant are provided with a canal which is connected with the rail during guiding and controlling their insertion into the disc space. The inconvenience of this solution is a lack of the possibility of positioning and anchoring the instrument during insertion of the implant into the intervertebral space. Moreover, there is a possibility that the instrument may slide and harm sensitive tissues.

A method of installation of an expandable prosthesis for support of the anterior spinal column is known from the instruction of use of the LfC implant. The method consists in:
- resection of vertebral body/bodies to enable secure, axial installation of the prosthesis,
- measurement of the resected space height,
- accurate selection of the prosthesis with regard to biomechanical and dimensional aspects corresponding to the size of the resected space,
- filling the prosthesis with an autogenous bone graft or another material enabling bone fusion,
- insertion of the prosthesis into surgically prepared space with the "Spacer grasper" in a way enabling a free approach to the blocking screw, paying special attention to a proper placement of external resistance rings with respect to the bearing surface of the vertebral body, so as not to irritate the spinal cord,
- extension of the prosthesis with the "Spacer dilator" to the required height, performing the desired distraction, and secure placement of the prosthesis between vertebral bodies,
- complementation of the inside of the prosthesis with an autogenous bone graft or anther material enabling bone fusion through special slots in sleeves,
- screwing down the blocking screw into a transverse notch of the inner sleeve so as to stabilize and securely block the mutual position of both sleeves of the spacer,
- releasing the instrument and withdrawing it from the surgical field.

A method of performance of vertebral body replacement surgery is known from the instruction of introducing the ECD implant - Expandable Corpectomy Device of Synthes The method consists in:
- performing corpectomy and cleaning vertebral endplates,
- spinal segment distraction for setting the anatomic height of intervertebral space,
- selection of implant's size,
- connecting the implant to the holding-distraction instrument by placing implant's teeth in implant's notches,
- placing the prosthesis in the resected part of the spine and aligning it in a sagittal and frontal plane, the optimal arrangement of the implant being the center of the vertebral endplate. To provide bone fusion, some space around vertebral endplates has to be provided,
- extension of the implant *in situ* with the holding-distraction instrument by rotating the handle of the instrument until the desired height of the implant is reached and the prosthesis anchors in vertebral endplates,
- releasing the implant from the instrument,
- filling the space around the implant, especially its anterior part with a bone graft or bone substitute.

The inconveniences of the above mentioned methods arise from their complexity and duration of surgery.

US 2008/0221694 A1 describes an interbody spacer system with a spacer body and an pivotable cylinder located in a recess of the body. The cylinder is pivotable about an axis which is parallel to a longitudinal axis of a central opening of the spacer. A tool can be screwed into a thread provided in the cylinder. Moreover the spacer is equipped with further holes extending substantially parallel to the longitudinal axis of the central opening.

WO 2007/124352 A2 describes methods of performing a spinal fusion procedure and a monorail instrument that protects the medial neural structures. The instrument includes a sliding platform for additional instrumentation and allows con-trolled delivery of various instruments for disc preparation and implant insertion. The monorail includes a tapered tip for easier insertion into a disc space and shoulders adjacent to the leading end that prevent the instrument from over extending into the disc space and damaging spinal tissue. The shoulder extends at about 90 degree relative to a guiding surface of the monorail.

Free of the inconveniences described above is the solution according to the present invention, wherein a prosthesis of the anterior spinal column is provided as defined in claim 1.

Further advantages are achieved by the embodiments indicated by the dependent claims.

Preferably, at least one end face or/and body's wall are provided with at least one pair of opposite guidelines. A part of at least one end face of the body located at the side of the manipulative hole is inclined at an angle α not greater than 80° relative to the transverse axis of the body.

The wall of the body from its internal side is preferably provided with a bar, within which a gap is made.

In one embodiment of the prosthesis, the wall of the body from its internal side is provided with a thread.

Inside the body, there is a positioned provided with overgrowth holes and at least one situating element, cooperating with at least one of the positioning holes made in the body. The positioner is provided with at least one hole, situated within the inside diameter of the body's manipulative hole, cooperating with the known installation instrument. The length of the body's manipulative hole corresponds to the distance between extreme locations of the positioner's hole, which is situated within the inside diameter of the manipulative hole. The positioner is in the form of a shaped solid, whose shape, depending on the embodiment, is comparable to a hollow barrel, a hollow cylinder, a bowl, or it is in the shape of polygon in a section.

Preferably, the positioner has got a longitudinal cut constituting spring arms, which facilitate the placement of the positioner inside the body. The situating element in variant embodiments is of the form of a pivot, projection, pin, a slot cooperating with an additional external fastening element, a hook or hump.

The positioner's situating element in one embodiment is of the length approximate to the positioner's length.

In an embodiment, the positioner's wall from its external side is provided with a thread cooperating with a thread situated on the body's wall from its internal side.

Preferably, the positioner is provided with a projection cooperating with a gap made in a body's threshold, securing the positioner against rotating within the body and falling out of it. Preferably, the hole in the positioner's wall is provided with a thread. In one embodiment, the positioner is provided with an internal protrusion, wherein the positioner's hole is situated. The positioner's hole, depending on the embodiment, is through or blind.

The prosthesis-guiding instrument is used for the proper installation of the prosthesis of the anterior spinal column. It is in the form of a rod ended on one side with a holder and on the other side with a shaped working element provided with a sliding segment. The working element in the instrument has got a guiding surface provided with at least one guide and a positioning surface situated on the opposite side of the working element, and a bearing surface situated with respect to the positioning surface at an angle β near or equal to 90° ; the bearing surface is provided with at least one anchoring element. Preferably, the guiding surface and positioning surface of the working element are situated relative to each other at an angle γ not greater than 20°.

The guiding surface of the working element is in variant embodiments convex or concave. A part of the working element provided with the guiding surface in one embodiment is in a section in the form comparable to a channel, wherein both lateral walls are provided with at least one guide situated in parallel to the positioning surface of the guiding instrument. The anchoring element of the guiding instrument is fastened to the bearing surface in a separable or inseparable manner. Preferably, the anchoring element is in the form of a spike.

Preferably, the bearing surface is provided with teeth.

The method of implantation of the prosthesis of the anterior spinal column in the intervertebral space consists in performing the following steps: After resection of the vertebral body, in thus surgically prepared intervertebral space, a distance between endplates of vertebrae adjacent to the resected vertebra is measured to select a prosthesis with a proper height, providing that the height of the prosthesis together with anchoring elements is at least equal to the distance between vertebral endplates of adjacent vertebral bodies. In the method, a prosthesis selected with regard to biomechanical and dimensional aspects is installed on the known installation instrument through the positioner's hole situated in the inside diameter of the body's manipulative hole, therewith the prosthesis is filled with a material enabling bone fusion. In the method, the guiding instrument is situated in the intervertebral space by resting its working element's bearing surface against the external surface of the vertebral body and placing the guiding instrument's anchoring element into this vertebral body to secure the proper location of the instrument and to restrict its mobility with respect to the vertebral body. Next, the installation instrument with the prosthesis installed on it is placed into the intervertebral space, resting the anchoring elements situated on the prosthesis end face unilaterally against the vertebral endplate opposite the vertebra, on which the guiding instrument is situated. Next, with rotational motion of the guiding instrument, the prosthesis is placed deep inside the intervertebral space along at least one guide of the guiding instrument cooperating with at least one pair of opposite guidelines made in the prosthesis body. Simultaneously, distraction of the resected space is performed using the guiding instrument. The support and rotation point is located on the anchoring elements situated on body's end face embedded in the vertebral body. Upon proper location of the prosthesis in the intervertebral space, the prosthesis is held in position by the installation instrument, and the guiding instrument is pulled out of the intervertebral space. Next, the installation instrument is disconnected from the prosthesis.

In an example of the method of implantation, after placing the prosthesis in the intervertebral space it is preferable to additionally support and fix it with an external stabilizer using fastening means screwed in the positioner's hole situated in the manipulative hole of the body.

The solution according to the invention increases precision of implantation, decreases the level of complexity of surgery, shortens the duration of surgery and improves its safety. The construction of the prosthesis allows for independent support of the anterior spinal column without the necessity of using additional stabilization, due to the prosthesis shape comparable to the shape of a vertebral body and owing to the fact that the prosthesis is made of a material with properties comparable to bone properties. The application of the internal positioner assures an attachment of the installation instrument during implantation through the positioner's hole located in the inside diameter of the body's manipulative hole. The prosthesis can also be connected with an additional stabilizer using a screw screwed in the positioner's hole situated in the inside diameter of the body's manipulative hole. The positioner is not in contact with the vertebral bones, thus it does not transfer the loads acting on the prosthesis. For the installation of the prosthesis it is sufficient to use only two instruments: the installation instrument and the guiding instrument, which in the procedure of installation assure distraction of the intervertebral space and precise placement of the prosthesis within the space. The construction of the guiding instrument assures accurate and secure introduction of the prosthesis into the intervertebral space. Service simplicity and ease of applying the instrumentation assure high precision and quickness of implantation, as well as an improvement in patient safety. A wide range of prosthesis' dimensions enables their application in most patients, assuring high ergonomics of surgeon's work.

Solutions according to the invention are presented in examples of embodiments, which do not restrict its range, in accompanying figures, where fig. 1 illustrates the body and the positioner in an arrangement, fig. 2 presents the body with a section and the positioner in an arrangement, fig. 3 illustrates the body with a section and the positioner situated within the body , the positioner being in the form of a hollowed cylinder provided with a thread on a part of the external surface, fig. 4 presents in a transverse section the body with the positioner situated within it, the positioner being in the form of a hollowed cylinder provided with two situating elements in the form of pivots, fig. 5 illustrates the body with a section, provided with a blind positioning hole and the positioner situated within the body in the form of a hollowed cylinder, fig. 6 presents in an arrangement the body provided with three elongated oblique manipulative holes and three elongated oblique positioning holes and the positioner in the form similar to a hollowed barrel, fig. 7 illustrates the body with a section and the positioner situated within the body, having the form of a hollowed cylinder, with the situating element in the form of a slot, fig. 8 presents the positioner in the form of a hollowed cylinder provided with a hump, fig. 9 presents the positioner in the form of a hollowed cylinder with the situating element in the form of a slot, fig. 10 illustrates the positioner in a form similar to a hollowed barrel, fig. 11 presents the positioner in the form of a hollowed cylinder, provided with a thread on a part of the wall from the external surface, fig. 12 presents the positioner in a form similar to a bowl having a transverse section of a polygon, fig. 13 presents the guiding instrument, fig. 14 illustrates the working element of the guiding instrument, fig. 15 presents the working element of the guiding instrument provided with a movable anchoring element in the form of a spike, fig. 16 presents the working element of the guiding instrument, whose part provided with a guiding surface in a section is in the shape comparable to a channel, fig. 17 presents the installation instrument, fig. 18 presents the guiding instrument with a convex guiding surface introduced into the intervertebral space, fig. 19 illustrates the method of prosthesis installation with the guiding instrument and the installation instrument, fig. 20 presents an illustration of the method of prosthesis installation with the use of the guiding instrument with a movable anchoring element in the form of a spike and the installation instrument, fig. 21 presents the working element of the guiding instrument with the prosthesis located on it, fig. 22 presents in a longitudinal section the prosthesis installed between two adjacent vertebrae reinforced with elements of external stabilization.

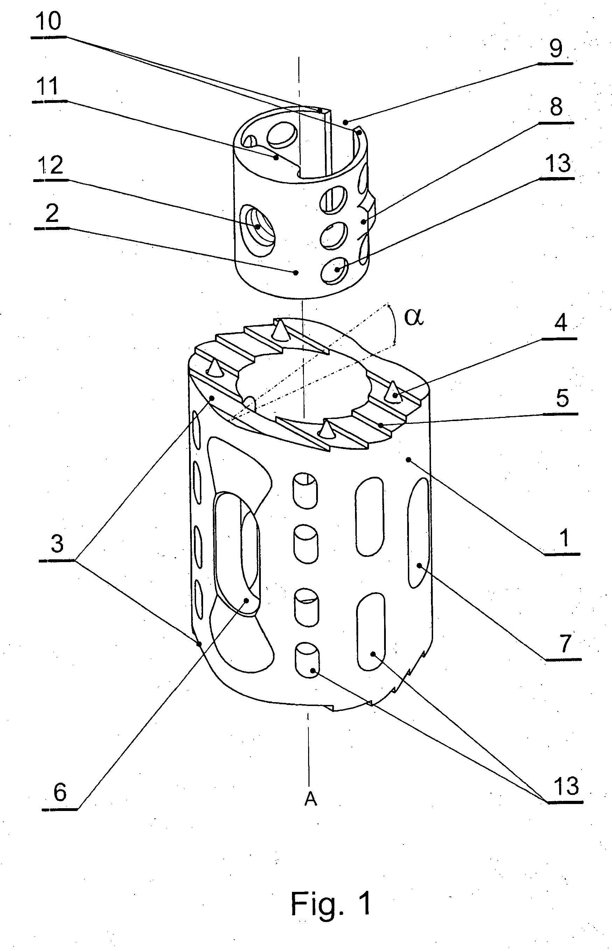

The prosthesis of the anterior spinal column presented in fig. 1 consists of a body 1 in the form of a perforated sleeve, and a positioner 2 cooperating with the body 1 and situated within. The body 1 is provided on every end face 3 with four anchoring elements 4 in the form of spikes and teeth 5. In the body's 1 wall corresponding to the anterior site of the spine, a through elongated manipulative hole 6 is made, and from the side of the manipulative hole 6 a through positioning hole 7 is located, whose longitudinal axis is parallel to the longitudinal axis of the manipulative hole 6. Longitudinal axes of the manipulative holes 6 and positioning hole 7 are parallel to the longitudinal axis A of the body 1. Each body's 1 end face 3 is cut from the side of the manipulative hole 6 and deflected from the transverse axis of body 1 at an angle α of 42°, which renders it possible in an initial phase of installation to embed and anchor the spikes 4 in the upper and lower vertebral endplates. The end face 3 of body 1 is inclined at an angle enabling the introduction of the prosthesis and fitting it to the patient's spine anatomy. The inside of the body in a section is in the shape of a wheel. The positioner 2 is in the form of a hollowed cylinder. On its external surface there is a situating element 8 in the form of a pivot situated in the positioning hole 7 of the body 1, limiting mobility of the positioner 2 inside the body 1. The positioner 2 is provided with a longitudinal cut 9 forming spring arms 10 and enabling their deflection for a more efficient introduction of the positioner 2 into the inside of the body 1. The positioner 2 is provided with an internal protrusion 11, where a threaded through hole 12 is made which is situated within the inside diameter of the body's 1 manipulative hole 6. Hole 12 enables the attachment of the end of the installation instrument, not visible in the figure, and this in turn improves the process of installing the prosthesis in the intervertebral space. In order to accelerate overgrowth of the prosthesis with bone tissue, both the wall of the body 1 and the positioner 2 are provided with overgrowth holes 13.

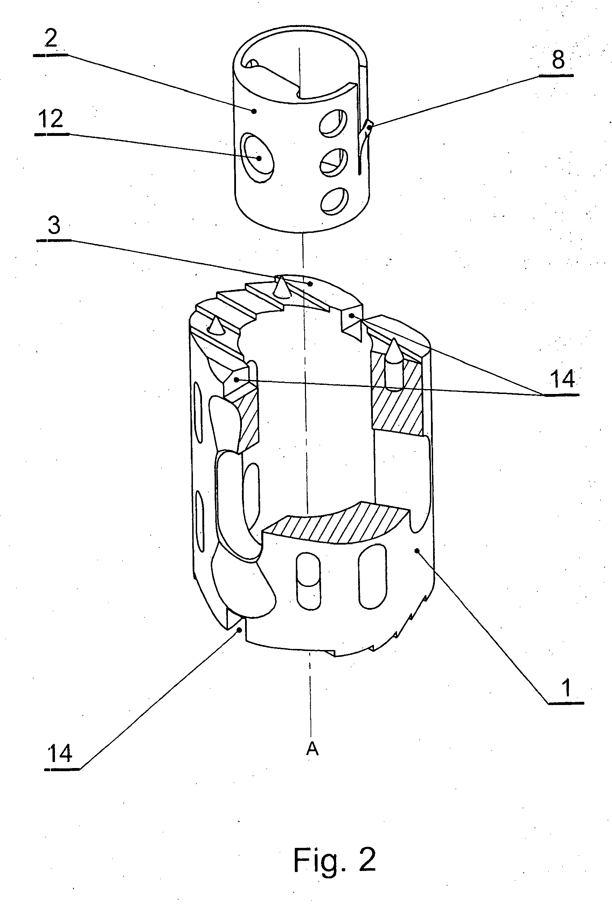

In one embodiment of the invention presented in fig. 2, each end face 3 of the body 1 is provided with a pair of opposite guidelines 14 cooperating with the guide of the guiding instrument, not visible in the figure. Positioner 2 is in the form of a hollowed, longitudinally cut cylinder provided with a situating element 8 in the form of a hook on a strip formed between the cuts. Hole 12 of the positioner 2 is non-threaded.

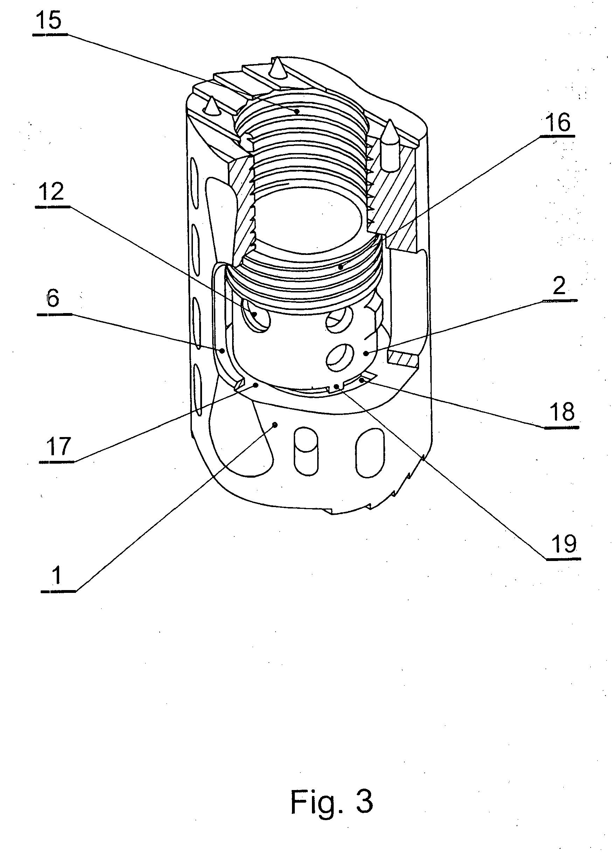

In another embodiment of the invention presented in fig. 3, a wall of the body 1 from its internal side is provided in a part of its length with a thread 15, whereas the positioner 2 situated inside the body 1 is provided on a part of its wall from its external side with a thread 16 cooperating with the thread 15 on body's 1 wall from its internal side. For preventing the positioner 2 from falling out of the body 1, the wall of the body 1 from its internal side is provided with a threshold 17, within which a gap 18 is made which cooperates with a projection 19 made on the wall of positioner 2 from its external side. The gap 18 prevents the positioner 2 from rotating inside the body 1 and facilitates placing hole 12 of positioner 2 within the inside diameter of the manipulative hole 6 of body 1.

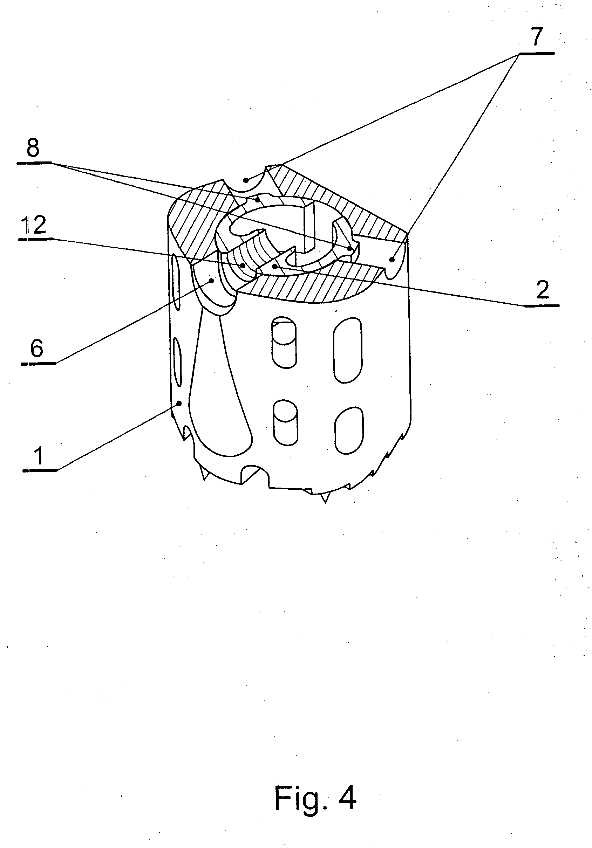

In another embodiment of the invention presented in fig. 4, the body 1 has got in its wall two through positioning holes 7 located on both sides of the manipulative hole 6. Manipulative hole 6 and positioning holes 7 are in the shape similar to a wheel. Positioner 2 cooperating with such body 1 is provided with two situating elements 8 in the form of pivots located on both sides of the hole 12 of positioner 2, which are situated in positioning holes 7 of the body 1.

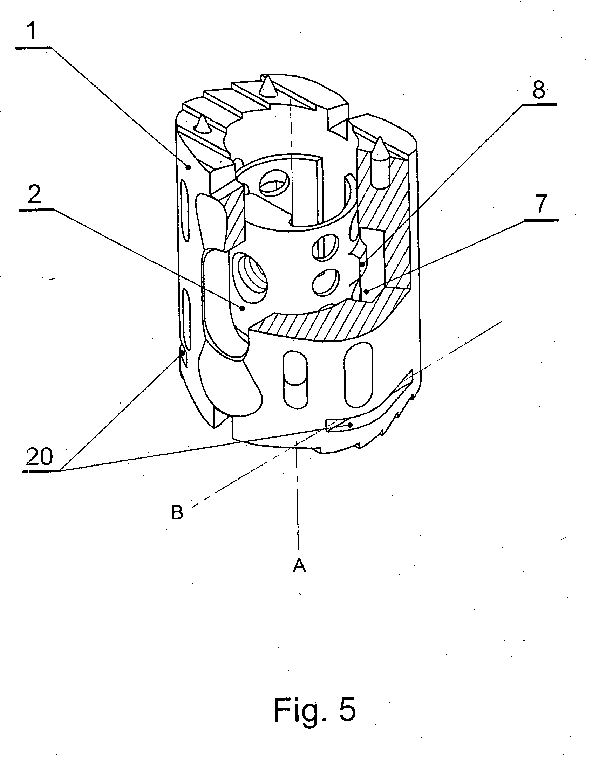

In still another embodiment of the invention presented in fig. 5, the positioner's 2 situating element 8 in the form of a pivot is situated in the blind elongated positioning hole 7 performed in body's 1 wall from its internal side. In the wall of body 1 , a pair of opposite guidelines 20 is made with longitudinal axes B lying in a plane perpendicular to the longitudinal axis A of the body 1.

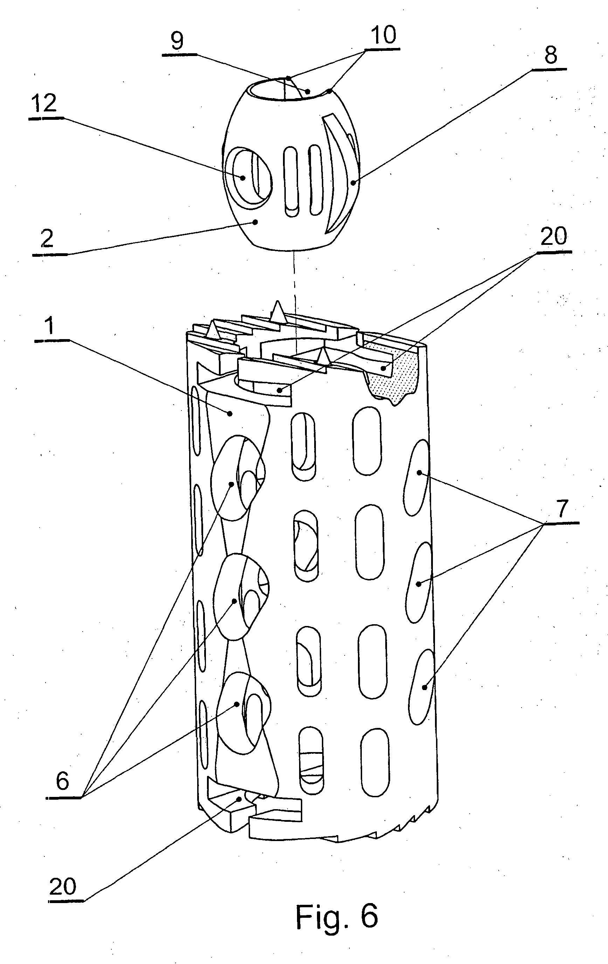

In another embodiment of the invention presented in fig. 6, the body 1 has got in its wall three through elongated oblique manipulative holes 6 and three elongated oblique through positioning holes 7. The wall of body 1 is provided with a pair of opposite guidelines 20 in the shape similar to the letter T. Positioner 2 is in the form similar to a hollowed barrel and is provided with a longitudinal cut 9 forming spring arms 10. Positioner 2 has got a non-threaded hole 12. Positioner 2 is equipped with a situating element 8 in the form of projection with a length corresponding approximately with positioner's 2 length, the situating element 8 is located on positioner's wall from its external side.

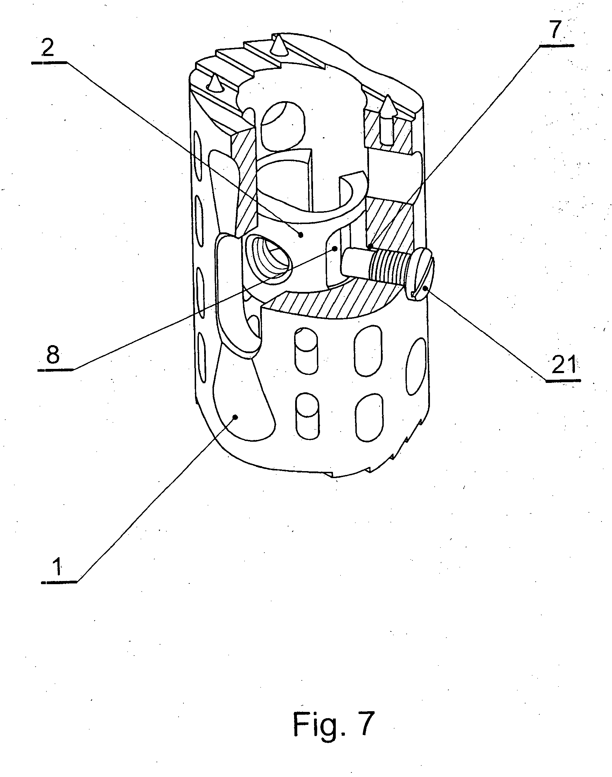

In another embodiment of the invention presented in fig. 7, the situating element 8 of positioner 2 is constituted by a through elongated slot for an additional fixing element 21 in the form of a screw situated in the positioning hole 7 of body 1

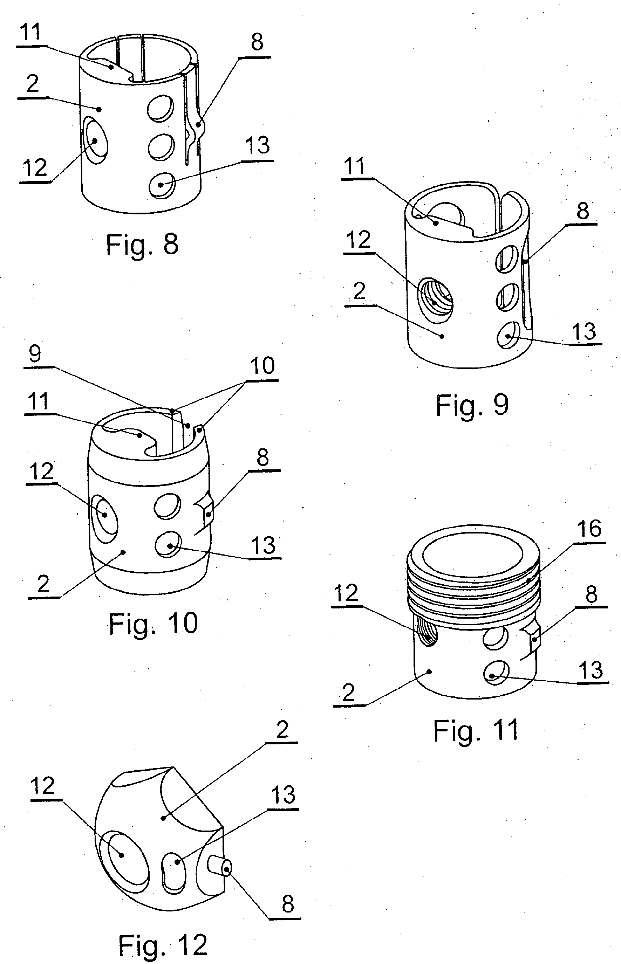

Figures 8 - 12 present further embodiments of the positioner. The positioner 2 presented in fig. 8 is in the form of a hollow, longitudinally cut cylinder provided with the situating element 8 in the form of a hump on the strip formed between cuts. The positioner 2 is provided with an inner thickened area 11, wherein a non-threaded through hole 12 is made: The positioner 2 shown in fig. 9 is in the form of a hollowed cylinder. The situating element 8 of the positioner 2 is constituted by an elongated through slot. The positioner 2 is provided with the inner thickened area 11, wherein a threaded through hole 12 is made. The positioner 2 presented in fig. 10 is in the form similar to a hollowed barrel. On its wall from the external side is located the situating element 8 in the form of a pivot. The positioner 2 is provided with a longitudinal cut 9 forming spring arms 10. Positioner 2 is provided with the inner thickened area 11, wherein the threaded through hole 12 is made. The positioner 2 shown in fig. 11 is in the form of a hollowed cylinder provided with a thread 16 in a part of the wall from its external side. The positioner 2 has got a through threaded hole 12, and the situating element 8 in the form of a pivot is located on its wall from the external side. In an embodiment presented in fig. 12, positioner 2 is in the form similar to a hollowed bowl ended with planes forming a polygon in a transverse section. The positioner 2 is provided with the non-threaded hole 12 and the situating element 8 in the form of a pin situated on positioner's 2 wall from its external side. For acceleration of prosthesis overgrowth with bone tissue; all embodiments of positioner 2 are provided with overgrowth holes 13.

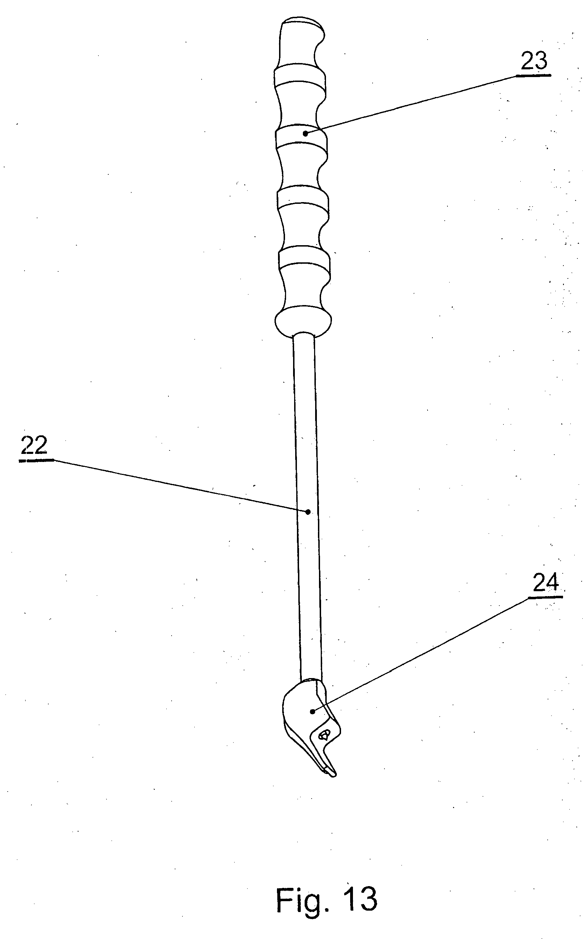

The guiding instrument presented in fig. 13 is in the form of a rod 22 ended with a handle 23 on one of the ends, and a working element 24 on the other end.

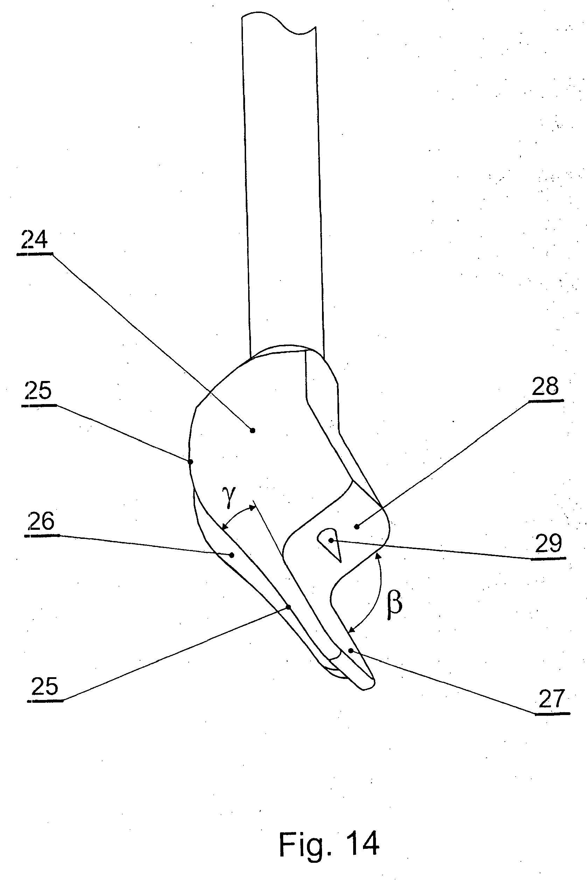

The working element 24 of the guiding instrument presented in fig. 14 has got a convex guiding surface 25, provided with a shaped guide 26, cooperating with guidelines 14, not visible in this figure, located in the end face 3 of the body 1. The working element 24 of the guiding instrument has got a positioning surface 27 and a bearing surface 28 situated relative to it at an angle β equal to 90°; the bearing surface 28 fixes the guiding instrument in a proper position relative to the vertebral body. The guiding surface 25 and positioning surface 27 of the working element 24 are situated relative to each other at an angle y equal to 10° forming a wedge. On the bearing surface 28 there is an immobile anchoring element 29 in the form of a spike, enabling anchoring of the guiding instrument in a bone of the vertebral body. The positioning surface 27 and bearing surface 28 are situated on the other side of the guiding surface 25.

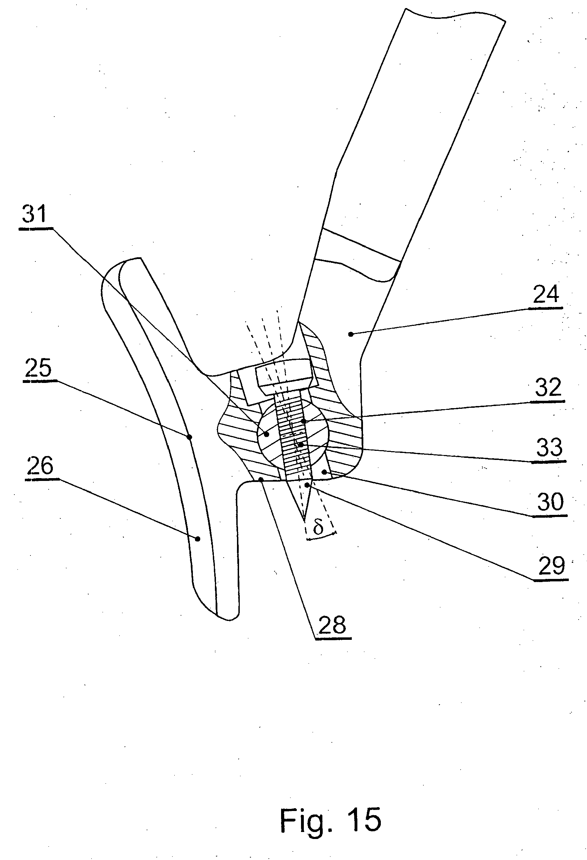

In a guiding instrument presented in fig. 15, the working element 24 of the guiding instrument has got a concave guiding surface 25 provided with a shaped guide 26, and the bearing surface 28 of the working element 24 is provided with a movable anchoring element 29 in the form of a spike. Within the working element 24 of the guiding instrument, in a plane approximately perpendicular to the bearing surface 28 an elongated port 30 is made, in which a rotationally movable cylinder 31 is located. The movable cylinder 31 has a threaded port 32 in its lateral wall. The port 32 enables screwing the anchoring element 29 into the cylinder 31. The anchoring element 29 is in the form of a spike and is provided with a thread 33 on the part located within the working element 24. It allows the regulation of the jut of the anchoring element 29 in the form of a spike from above the bearing surface 28 of the working element 24. The anchoring element 29 in the form of a spike may move in a swinging way within the inside diameter of the elongated through port 30 due to rotational movement of the cylinder 31 in a range of angle δ changes of ±4°. This movement takes place only in one plane.

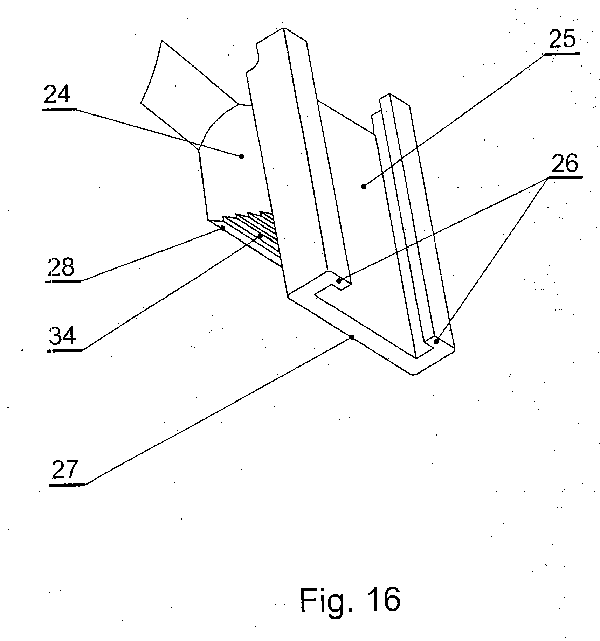

In a guiding instrument shown in fig. 16, a part of the working element 24 provided with the guiding surface 25 in a section is in the form comparable to a channel. The guides 26 are situated on the edges of both walls in parallel to the positioning surface 27. The bearing surface 28 is provided with teeth 34 preventing the bearing surface 28 from uncontrolled move along the vertebral body.

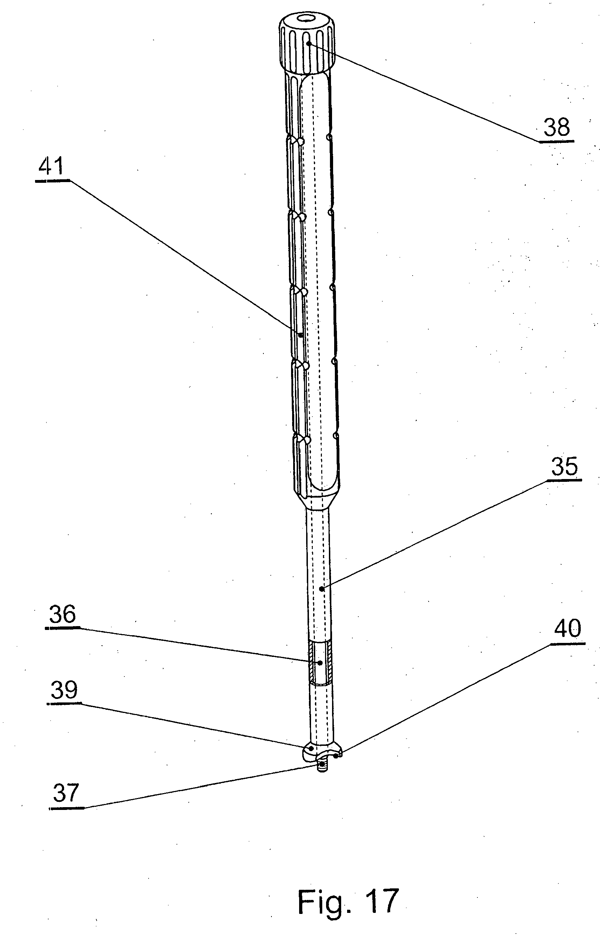

The installation instrument presented in fig. 17 is constituted by a sleeve 35, within which a tang 36 is located, ended on the one side with a junction 37 enabling provisional connection with the hole 12 in the positioner 2, not visible in the figure, and on the other side with a knob 38 cooperating with the junction 37. The end of the sleeve from the side of the junction 37 is provided with a collar 39 with a bearing plane 40. The bearing plane 40 of the collar 39 has got a profile similar to the shape of the prosthesis body's 1, which allows precise adjustment of the instrument to body 1 of the prosthesis, which is not visible in the figure, and prevents the body 1 from moving relative to the installation instrument, thus improving precision of prosthesis installation. A holder 41 is mounted on the sleeve 35 underneath the knob 38.

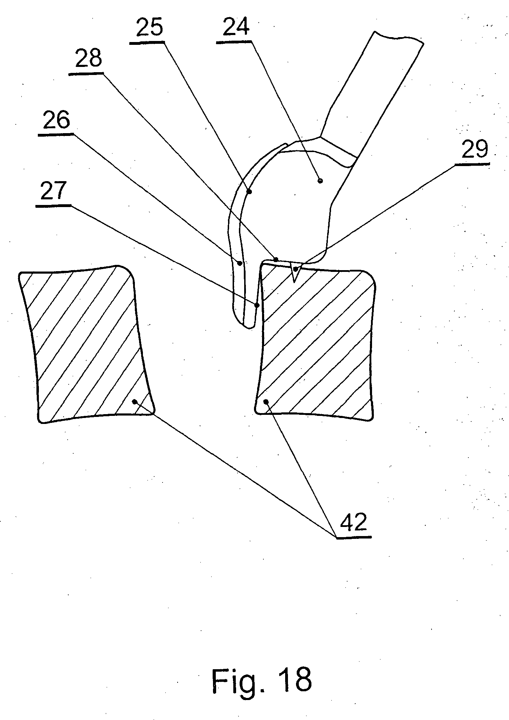

Fig. 18 illustrates the guiding instrument introduced into the intervertebral space. The working element 24 of the guiding instrument has got a convex guiding surface 25, provided with a shaped guide 26 and positioning surface 27 and a bearing surface 28 situated relative to the positioning surface 27 at an angle β equal to 90°. The positioning surface 27 and bearing surface 28 are situated on the opposite side of the guiding surface 25. On the bearing surface 28 is located an immobile anchoring element 29 in the form of a spike. The bearing surface 28 of the guiding instrument is rested against the external part of the vertebral body 42, within which the immobile anchoring element 29 in the form of a spike is situated.

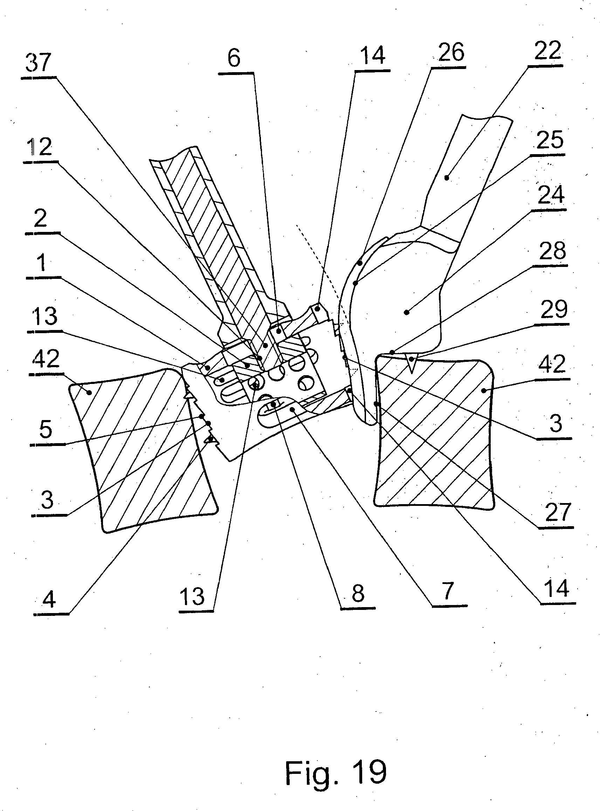

The method of installation of the prosthesis of the anterior spinal column is illustrated in fig. 19. After resection of the vertebral body, in thus surgically prepared intervertebral space, a distance between endplates of vertebral bodies 42 adjacent to the resected vertebra is measured to select a prosthesis with a proper height, providing that the height of the prosthesis together with anchoring elements 4 is at least equal to the distance between endplates of adjacent vertebral bodies 42. The prosthesis, selected with regard to biomechanical and dimensional aspects, for installation in the intervertebral space consists of a sleeve body 1 whose each end face 3 is provided with four anchoring elements 4 in the form of spikes, teeth 5 and a pair of opposite guidelines 14; the sleeve body 1 has in its wall a through elongated manipulative hole 6, and a through elongated positioning hole 7 and a positioner 2, situated inside the body 1, in the shape of a hollowed cylinder provided with a threaded hole 12, situated within the inside diameter of the body's 1 manipulative hole 6 and a situating element 8 in the form of a pivot located in the positioning hole 7 of the body 1, and also a longitudinal cut 9, not visible in the figure, which forms spring arms 10. Both the body 1 and positioner 2 are provided with overgrowth holes 13 for filling the prosthesis with a material enabling bone overgrowth. The prosthesis that has been selected is installed on a junction 37 of the installation instrument through a threaded positioner's 2 hole 12 situated in body's 1 elongated manipulative hole 6. Next, the prosthesis is filled with a material enabling bone fusion, using a mallet of bony fragments. Then the guiding instrument is situated in the intervertebral space. The guiding instrument is in the form of a rod 22 ended with a handle 23 on the one side , not visible in the figure, and a working element 24 on the other side. The working element 24 of the guiding instrument has got a convex guiding surface 25, provided with a shaped guide 26, cooperating with a pair of opposite guidelines 14 located in the end face 3 of body 1. The working element 24 of the guiding instrument has got a positioning surface 27 and a bearing surface 28 situated relative to the positioning surface at an angle β equal to 90°. The bearing surface 28 fixes the guiding instrument in a proper position with respect to the vertebral body. On the bearing surface 28 is located an immobile anchoring element 29 in the form of a spike. The positioning surface 27 and bearing surface 28 are situated on an opposite side of the guiding surface 25. The guiding instrument is introduced to the intervertebral space by resting its bearing surface 28 against the external part of the vertebral body 42 and the guiding instrument's immobile anchoring element 29 in the form of a spike is placed in this vertebral body in order to place the guiding instrument properly and restrict its mobility relative to the vertebral body 42. Next, the installation instrument with the prosthesis installed on it is placed in the intervertebral space by unilaterally resting the anchoring elements 4 in the form of spikes situated on the end face 3 of body 1 against the endplate of a vertebra 42 opposite the vertebra 42, on which the guiding instrument is placed. Next, with rotational movement of the guiding instrument the prosthesis is positioned deep inside the intervertebral space along the guide 26 of the guiding instrument cooperating with a pair of opposite guidelines 14 performed in the end face 3 of the prosthesis body 1, the intervertebral space is simultaneously distracted with the use of the guiding instrument, where the support and rotation point is located on the anchoring elements 4 in the form of spikes situated on the end face 3 of body 1 embedded in the endplate of the vertebral body 42. After placement, the prosthesis is held in position by means of the installation instrument and the guiding instrument is removed from the intervertebral space. Next, the installation instrument is disconnected from the prosthesis.

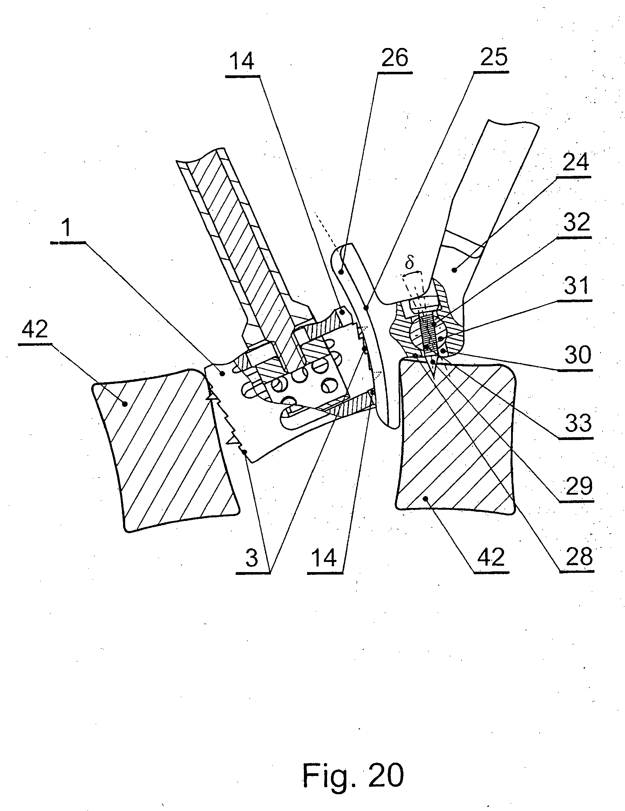

Fig. 20 shows an example of installation of the prosthesis with the use of the guiding instrument, whose working element 24 has got a concave guiding surface 25 provided with a shaped guide 26 cooperating with a pair of opposite guidelines 14 performed in the end face 3 of body 1. A movable anchoring element 29 in the form of a spike is located on the bearing surface 28 of the guiding instrument. Within the working element 24 of the guiding instrument, in a plane approximately perpendicular to the guiding surface 28 there is an elongated port 30, within which a rotationally movable cylinder 31 is located, having in its lateral wall a threaded port 32. The port 32 enables screwing the anchoring element 29 in the form of a spike into the cylinder 31 . The anchoring element is provided with a thread 33 on the part located within the working element 24.

It allows the regulation of jut of the anchoring element 29 in the form of a spike from above the bearing surface 28 of the working element 24. The anchoring element 29 in the form of a spike may move in a swinging way within the inside diameter of the elongated through port 30 due to rotational movement of the cylinder 31 in a range of angle δ changes of ±4°. This movement takes place only in one plane. The guiding instrument is introduced to the intervertebral space by resting its bearing surface 28 against the external part of the vertebral body 42 and the movable anchoring element 29 in the form of a spike is placed into this vertebral body 42. Next, the installation instrument with the prosthesis installed on it is positioned in the intervertebral space by unilaterally resting the anchoring elements 4 in the form of spikes situated on the end face 3 of body 1 against the endplate of the vertebra 42 opposite the vertebra 42, on which the guiding instrument is placed. Next, the guiding surface 25 of the working element 24 of the guiding instrument is deflected from the vertebral body 42 and the placement of the prosthesis into the intervertebral space starts along the guide 26 of the guiding instrument cooperating with a pair of opposite guidelines 14 located in the end face 3 of body 1. Next, the guiding surface 25 of the guiding instrument's working element 24 is inclined towards the vertebral body 42 and the prosthesis is situated deep inside the intervertebral space. Deflection of the guiding surface 25 of the guiding instrument's working element 24 from the vertebral body 42, and its further inclination towards the vertebral body 42 is possible due to the swinging movement of the anchoring element in the form of a spike in a range of ±4°.

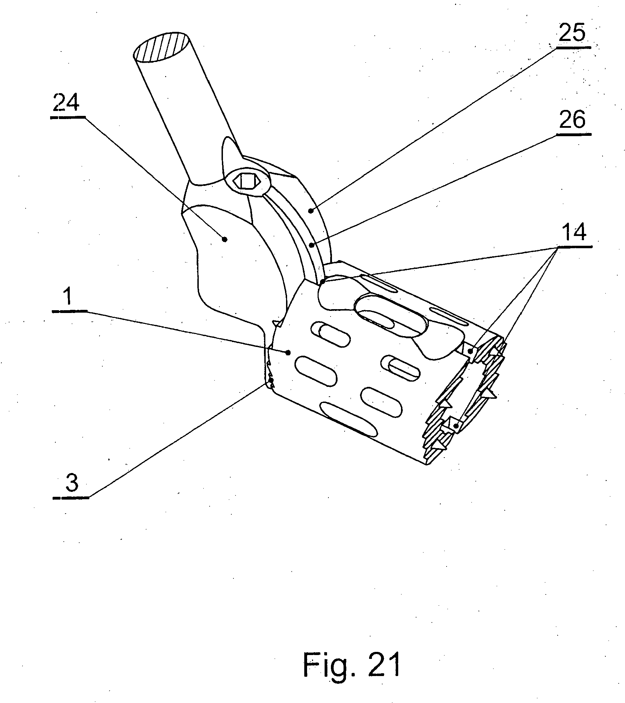

Fig. 21 illustrates the guiding instrument, whose working element 24 has got a convex guiding surface 25 provided with a shaped guide 26, cooperating with a pair of opposite guidelines 14 located in end faces 3 of body 1.

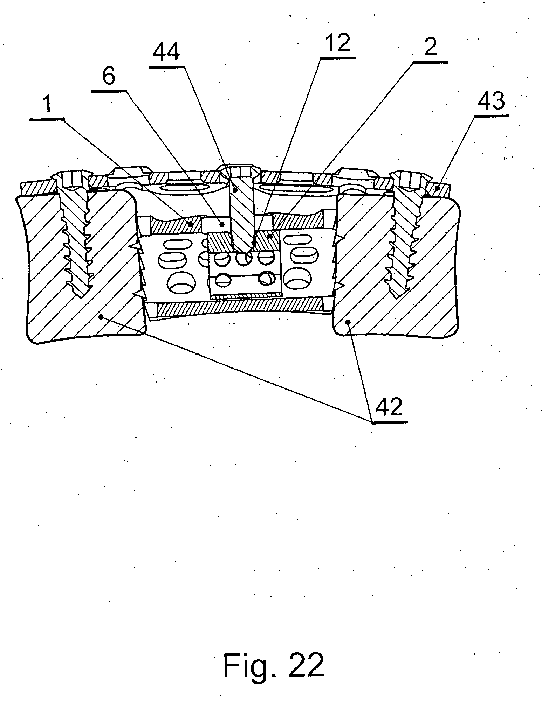

In an example of implantation of the prosthesis shown in fig. 22, after placing the prosthesis in the intervertebral space, the prosthesis is additionally fixed with an external plate stabilizer 43 using a fixing element 44 in the form of a screw driven into the positioner's 2 hole 12 situated in the manipulative hole 6 of body 1.

## Claims

1. The prosthesis of the anterior spinal column containing:
- a perforated sleeve body with a longitudinal axis (A) and a circumferential body wall encircling a space extending along the longitudinal axis (A), where the sleeve body is provided with overgrowth holes in its body wall and at least one anchoring element and teeth on each end face of the body's surface, where the sleeve body (1) has got in its body wall at least one elongated through manipulative hole (6) and at least one elongated positioning hole (7) cooperating with it, whose longitudinal axes are parallel relative to each other, and
- a positioner (2) situated within the space encircled by the body wall and being movable along the longitudinal axis A of the sleeve body (1), the positioner (2) being provided with overgrowth holes (13) and at least one situating element (8) cooperating with the at least one body's (1) positioning hole (7) and the positioner (2) being provided with at least one hole (12) which can be aligned with the manipulative hole (6), where the cooperation of the at least one situating element (8) and the positioning hole (7) limits the mobility of the positioner (2) inside the sleeve body (1),
where
the length of body's (1) manipulative hole (6) corresponds with the distance between extreme locations of positioner's (2) hole (12) that are allowed by the cooperation of the at least one situating element (8) and the positioning hole (7) such that the positioner's (2) hole (12) is situated within the inside diameter of the manipulative hole (6).

2. The prosthesis according to claim 1 is **characterized in that** at least one end face (3) of the body (1) is provided with at least one pair of opposite guidelines (14).

3. The prosthesis according to any of the claims 1-2 is **characterized in that** a part of at least one end face (3) of body (1) located at the side of the manipulative hole (6) is deflected from the transverse axis of body (1) at an angle not greater than 80°.

4. The prosthesis according to any of the claims 1-3 is **characterized in that** the wall of body (1) is provided with at least one pair of opposite guidelines (20).

5. The prosthesis according to any of the claims 1-4 is **characterized in that** the positioning hole (7) is through or the positioning hole (7) is blind and made in the wall of body (1) from its internal side.

6. The prosthesis according to any of the claims 1-5 is **characterized in that** the wall of body (1) from its internal side is preferably provided with a threshold (17) and/or with a thread (15).

7. The prosthesis according to claim 7 is **characterized in that** the threshold (17) is provided with a gap (18).

8. The prosthesis according to any of the claims 1-7 is **characterized in that** the positioner (2) is in the form of a shaped solid where the positioner (2) constitutes a solid in a form similar to a hollowed barrel or the positioner (2) is in the shape of a hollowed cylinder or the positioner (2) is in the shape of a bowl or the positioner (2) in a transverse section is in the shape of a polygon.

9. The prosthesis according to any of the claims 1-8 is **characterized in that** there is a longitudinal cut (9) in the positioner (2) forming spring arms (10).

10. The prosthesis according to any of the claims 1-9 **characterized in that** the situating element (8) of the positioner (2) is constituted by a pivot or a projection constitutes the situating element (8) of the positioner (2) or a pin constitutes the situating element (8) of the positioner (2) or a hole cooperating with an additional fastening element (21) constitutes the situating element (8) of the positioner (2).

11. The prosthesis according to any of the claims 1-10 is **characterized in that** the situating element (8) of the positioner (2) is of a length comparable to the length of the positioner (2).

12. The prosthesis according to any of the claims 1-11 is **characterized in that** the wall of the positioner (2) from its external side is provided with a thread (16) cooperating with the thread (15) situated on body's (1) wall from its internal side.

13. The prosthesis according to any of the claims 1-12 is **characterized in that** the positioner (2) is provided with a projection (19) cooperating with a gap (18) made in the threshold (17) of body (1).

14. The prosthesis according to any of the claims 1-13 is **characterized in that** the hole (12) of positioner (2) is provided with a thread.

15. The prosthesis according to any of the claims 1-14 is **characterized in that** the positioner (2) is provided with an inner protrusion (11), wherein the hole (12) of positioner (2) is situated.

## Patentansprüche

1. Prothese der vorderen Wirbelsäule, umfassend:
- einen durchlöchertern Hülsenkörper mit einer Längsachse (A) und einer umlaufenden Körperwand, die einen Raum umgibt, der sich entlang der Längsachse (A) erstreckt, wobei der Hülsenkörper mit Überwachsungsöffnungen in seiner Körperwand und mindestens einem Verankerungselement und Zähnen auf jeder Stirnfläche der Oberfläche des Körpers ausgestattet ist, wobei der Hülsenkörper (1) in seiner Körperwand mindestens eine längliche durchgehende Manipulationsöffnung (6) und mindestens eine damit zusammenwirkende längliche Positionierungsöffnung (7) aufweist, deren Längsachsen parallel zueinander liegen, und
- einen Positionierer (2), der in dem Raum liegt, der von der Körperwand umgeben wird, und der entlang der Längsachse (A) des Hülsenkörpers (1) beweglich ist, wobei der Positionierer (2) mit Überwachsungsöffnungen (13) und mindestens einem Ausrichtungselement (8), das mit der mindestens einen Positionierungsöffnung (7) des Körpers (1) zusammenwirkt, ausgestattet ist, und der Positionierer (2) mit mindestens einer Öffnung (12), die auf die Manipulationsöffnung (6) ausgerichtet werden kann, ausgestattet ist, wobei das Zusammenwirken des mindestens einen Ausrichtungselements (8) und der Positionierungsöffnung (7) die Beweglichkeit des Positionierers (2) innerhalb des Hülsenkörpers (1) begrenzt,
wobei
die Länge der Manipulationsöffnung (6) des Körpers (1) mit dem Abstand zwischen Extrempositionen der Öffnung (12) des Positionierers (2), die durch das Zusammenwirken des mindestens einen Ausrichtungselements (8) mit der Positionierungsöffnung (7) zugelassen werden, korrespondiert, sodass die Öffnung (12) des Positionierers (2) innerhalb des inneren Durchmessers der Manipulationsöffnung (6) liegt.

2. Prothese nach Anspruch 1, **dadurch gekennzeichnet, dass** mindestens eine Stirnfläche (3) des Körpers (1) mit mindestens einem Paar einander gegenüberliegender Führungen (14) ausgestattet ist.

3. Prothese nach einem der Ansprüche 1 - 2, **dadurch gekennzeichnet, dass** ein Teil mindestens einer Stirnfläche (3) des Körpers (1), die an der Seite der Manipulationsöffnung (6) liegt, mit einem Winkel von nicht mehr als 80° zur Querachse des Körpers (1) geneigt ist.

4. Prothese nach einem der Ansprüche 1 - 3, **dadurch gekennzeichnet, dass** die Wand des Körpers (1) mit mindestens einem Paar einander gegenüberliegender Führungen (20) ausgestattet ist.

5. Prothese nach einem der Ansprüche 1 - 4, **dadurch gekennzeichnet, dass** die Positionierungsöffnung (7) durchgehend ist oder die Positionierungsöffnung (7) blind ist und auf der Innenseite der Wand des Körpers (1) ausgebildet ist.

6. Prothese nach einem der Ansprüche 1 - 5, **dadurch gekennzeichnet, dass** die Wand des Körpers (1) auf seiner Innenseite vorzugsweise eine Schwelle (17) und/oder ein Gewinde (15) aufweist.

7. Prothese nach Anspruch 7, **dadurch gekennzeichnet, dass** die Schwelle (17) eine Aussparung (18) aufweist.

8. Prothese nach einem der Ansprüche 1 - 7, **dadurch gekennzeichnet, dass** der Positionierer (2) die Form eines geformten Festkörpers aufweist, wobei der Positionierer (2) einen Festkörper in Form ähnlich einer ausgehöhlten Tonne aufweist, oder der Positionierer (2) die Form eines ausgehöhlten Zylinders aufweist, oder der Positionierer (2) die Form einer Schale aufweist, oder der Positionierer (2) einen Querschnitt in Form eines Polygons aufweist.

9. Prothese nach einem der Ansprüche 1 - 8, **dadurch gekennzeichnet, dass** ein Längsschlitz (9) im Positionierer (2) vorhanden ist, der Federarme (10) bildet.

10. Prothese nach einem der Ansprüche 1 - 9, **dadurch gekennzeichnet, dass** das Ausrichtungselement (8) des Positionierers (2) von einem Zapfen gebildet wird, oder ein Vorsprung bildet das Ausrichtungselement (8) des Positionierers (2), oder ein Stift bildet das Ausrichtungselement (8) des Positionierers (2), oder eine Öffnung, die mit einem zusätzlichen Befestigungselement (21) zusammenwirkt, bildet das Ausrichtungselement (8) des Positionierers (2).

11. Prothese nach einem der Ansprüche 1 - 10, **dadurch gekennzeichnet, dass** das Ausrichtungselement (8) des Positionierers (2) eine Länge vergleichbar zur Länge des Positionierers (2) aufweist.

12. Prothese nach einem der Ansprüche 1 - 11, **dadurch gekennzeichnet, dass** die Wand des Positionierers (2) auf seiner Außenseite mit einem Gewinde (16) ausgestattet ist, das mit dem Gewinde (15), das auf der Innenseite der Wand des Körpers (1) liegt, zusammenwirkt.

13. Prothese nach einem der Ansprüche 1 - 12, **dadurch gekennzeichnet, dass** der Positionierer (2) einen Vorsprung (19) aufweist, der mit einer Aussparung (18), die in der Schwelle (17) des Körpers (1) liegt, zusammenwirkt.

14. Prothese nach einem der Ansprüche 1 - 13, **dadurch gekennzeichnet, dass** die Öffnung (12) des Positionierers (2) ein Gewinde aufweist.

15. Prothese nach einem der Ansprüche 1 - 14, **dadurch gekennzeichnet, dass** der Positionierer (2) einen inneren Vorsprung (11) aufweist, in dem die Öffnung (12) des Positionierers (2) liegt.

## Revendications

1. Prothèse de la colonne vertébrale antérieure contenant :
- un corps de type manchon perforé ayant un axe longitudinal (A) et une paroi de corps circonférentielle encerclant un espace s'étendant le long de l'axe longitudinal (A), le corps de type manchon étant pourvu de trous de surcroissance dans sa paroi de corps et d'au moins un élément d'ancrage et de dents sur chaque face d'extrémité de la surface de son corps, le corps de type manchon (1) ayant dans sa paroi de corps au moins un trou de manipulation débouchant allongé (6) et au moins un trou de positionnement allongé (7) coopérant avec lui, dont les axes longitudinaux sont parallèles entre eux, et
- un positionneur (2) situé dans l'espace encerclé par la paroi de corps et mobile le long de l'axe longitudinal A du corps de type manchon (1), le positionneur (2) étant pourvu de trous de surcroissance (13) et d'au moins un élément de localisation (8) coopérant avec l'au moins un trou de positionnement (7) du corps (1) et le positionneur (2) étant pourvu d'au moins un trou (12) qui peut être aligné avec le trou de manipulation (6), la coopération de l'au moins un trou de localisation (8) et du trou de positionnement (7) limitant la mobilité du positionneur (2) à l'intérieur du corps de type manchon (1),
la longueur du trou de manipulation (6) du corps (1) correspondant à la distance entre les positions extrêmes du trou (12) du positionneur (2) qui sont autorisées par la coopération de l'au moins un élément de localisation (8) et du trou de positionnement (7), de telle sorte que le trou (12) du positionneur (2) soit situé dans le diamètre intérieur du trou de manipulation (6).

2. Prothèse selon la revendication 1, **caractérisée en ce qu'**au moins une face d'extrémité (3) du corps (1) est pourvue d'au moins une paire de lignes de guidage (14) en regard l'une de l'autre.

3. Prothèse selon l'une quelconque des revendications 1 à 2, **caractérisée en ce qu'**une partie d'au moins une face d'extrémité (3) du corps (1) située du côté du trou de manipulation (6) est déviée de l'axe transversal du corps (1) selon un angle non supérieur à 80°.

4. Prothèse selon l'une quelconque des revendications 1 à 3, **caractérisée en ce que** la paroi du corps (1) est pourvue d'au moins une paire de lignes de guidage (20) en regard l'une de l'autre.

5. Prothèse selon l'une quelconque des revendications 1 à 4, **caractérisée en ce que** le trou de positionnement (7) est débouchant ou bien le trou de positionnement (7) est borgne et formé dans la paroi du corps (1), sur sa face interne.

6. Prothèse selon l'une quelconque des revendications 1 à 5, **caractérisée en ce que** la paroi du corps (1) est de préférence pourvue, sur sa face interne, d'un seuil (17) et/ou d'un filetage (15).

7. Prothèse selon la revendication 7, **caractérisée en ce que** le seuil (17) est pourvu d'un interstice (18).

8. Prothèse selon l'une quelconque des revendications 1 à 7, **caractérisée en ce que** le positionneur (2) se présente sous la forme d'un solide façonné où le positionneur (2) constitue un solide ayant une forme similaire à un baril creux, ou bien le positionneur (2) se présente sous la forme d'un cylindre creux, ou bien le positionneur (2) se présente sous la forme d'un bol, ou encore le positionneur (2) a une section transversale de forme polygonale.

9. Prothèse selon l'une quelconque des revendications 1 à 8, **caractérisée en ce qu'**il y a dans le positionneur (2) une découpe longitudinale (9) formant des bras de ressort (10).

10. Prothèse selon l'une quelconque des revendications 1 à 9, **caractérisée en ce que** l'élément de localisation (8) du positionneur (2) est constitué d'un pivot ou bien une partie saillante constitue l'élément de localisation (8) du positionneur (2), ou bien une broche constitue l'élément de localisation (8) du positionneur (2), ou encore un trou coopérant avec un élément de fixation additionnel (21) constitue l'élément de localisation (8) du positionneur (2).

11. Prothèse selon l'une quelconque des revendications 1 à 10, **caractérisée en ce que** l'élément de localisation (8) du positionneur (2) a une longueur comparable à la longueur du positionneur (2).

12. Prothèse selon l'une quelconque des revendications 1 à 11, **caractérisée en ce que** la paroi du positionneur (2) est pourvue, sur sa face externe, d'un filetage (16) coopérant avec le filetage (15) situé sur la face interne de la paroi du corps (1).

13. Prothèse selon l'une quelconque des revendications 1 à 12, **caractérisée en ce que** le positionneur (2) est pourvu d'une partie saillante (19) coopérant avec un interstice (18) formé dans le seuil (17) du corps (1).

14. Prothèse selon l'une quelconque des revendications 1 à 13, **caractérisée en ce que** le trou (12) du positionneur (2) est pourvu d'un filetage.

15. Prothèse selon l'une quelconque des revendications 1 à 14, **caractérisée en ce que** le positionneur (2) est pourvu d'une partie saillante interne (11) dans laquelle est situé le trou (12) du positionneur (2).
